Europäisches Patentamt

European Patent Office

Office européen des brevets

(11) Veröffentlichungsnummer: **0 125 514**
A2

(12) **EUROPÄISCHE PATENTANMELDUNG**

(21) Anmeldenummer: **84104244.3**

(22) Anmeldetag: **14.04.84**

(51) Int. Cl.³: **C 07 G 7/00**
G 01 N 33/54, A 61 K 39/395

(30) Priorität: **20.04.83 DE 3314293**

(43) Veröffentlichungstag der Anmeldung:
**21.11.84 Patentblatt 84/47**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Bohn, Hans, Dr.**
**Oberer Eichweg 26**
**D-3550 Marburg(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) Membran-assoziiertes Plazentaprotein (MP1), Verfahren zu seiner Gewinnung sowie seine Verwendung.

(57) Es wird ein membran-assoziiertes Plazentaprotein, genannt MP₁, sowie ein Verfahren zu seiner Gewinnung beschrieben. Das Protein MP₁ besitzt die Parameter:

a) eine elektrophoretische Beweglichkeit im Bereich zwischen $\alpha_2$ und $\beta_1$ Globulinen;

b) einen isoelektrischen Punkt von 4,75 ± 0,15;

c) einen Sedimentationskoeffizienten $S^{\circ}_{20,w}$ von 6,65 ± 0,15 S;

d) ein im Natriumdodecylsulfat (SDS)-haltigen Polyacrylamidgel bestimmtes Molekulargewicht von 180.000 ± 20.000, wobei die Moleküle von MP₁ aus anscheinend identischen Untereinheiten, die ein Molekulargewicht von 90.000 ± 10.000 haben und nicht-kovalent zusammengehalten werden, aufgebaut sind;

c) einen Extinktionskoeffizienten $E^{1\%}_{1cm}$ (280 nm) von 8,2 ± 0,4;

f) einen Kohlenhydratanteil von 9,6 ± 2.5% (Mannose 2,2 ± 0,5%, Galaktose 1,7 ± 0,4%, Fucose 0,4 ± 0,2%, N-Acetyl-Glukosamin 3,2 ± 0,8%, N-Acetyl-Neuraminsäure 2,1 ± 0,6%) und

g) eine bestimmte Aminosäurenzusammensetzung.

MP₁ kann verwendet werden, um Antiseren herzustellen, die dazu dienen können, MP₁ nachzuweisen und zu bestimmen. Weiterhin können Antikörper gegen MP₁ zur radioimmunologischen Lokalisation und zur immuntherapeutischen Behandlung von Tumoren, die MP₁ produzieren, eingesetzt werden.

Membran-assoziiertes Plazentaprotein ($MP_1$), Verfahren
zu seiner Gewinnung sowie seine Verwendung

Die Erfindung betrifft ein membran-assoziiertes Plazentaprotein, genannt $MP_1$, sowie ein Verfahren zu seiner
Gewinnung. $MP_1$ kann verwendet werden, um Antiseren herzustellen, die dazu dienen können, $MP_1$ nachzuweisen und
zu bestimmen. Weiterhin können Antikörper gegen $MP_1$ zur
radioimmunologischen Lokalisation und zur immuntherapeutischen Behandlung von Tumoren, die $MP_1$ produzieren,
eingesetzt werden.

Gegenstand der Erfindung ist das Protein $MP_1$, gekennzeichnet durch

a) eine elektrophoretische Beweglichkeit im Bereich
   zwischen $\alpha_2$ und $\beta_1$ Globulinen;

b) einen isoelektrischen Punkt von $4,75 \pm 0,15$;

c) einen Sedimentationskoeffizienten $S^\circ_{20,w}$ von $6,65 \pm 0,15$ S;

d) ein im Natriumdodecylsulfat (SDS)-haltigen Polyacrylamidgel bestimmtes Molekulargewicht von $180.000 \pm 20.000$, wobei die Moleküle von $MP_1$ aus anscheinend
   identischen Untereinheiten, die ein Molekulargewicht
   von $90.000 \pm 10.000$ haben und nicht-kovalent zusammengehalten werden, aufgebaut sind;

e) einen Extinktionskoeffizienten $E^{1\%}_{1cm}$ (280 nm) von $8,2 \pm 0,4$;

f) einen Kohlenhydratanteil von $9,6 \pm 2,5\%$ (Mannose $2,2 \pm 0,5\%$, Galaktose $1,7 \pm 0,4\%$, Fucose $0,4 \pm 0,2\%$, N-
   Acetyl-Glukosamin $3,2 \pm 0,8\%$, N-Acetyl-Neuraminsäure
   $2,1 \pm 0,6\%$) und

g) die folgende Aminosäurenzusammensetzung:

| Aminosäure | Reste pro 100 Reste (Mol %) | Variations-koeffizient |
|---|---|---|
| Cystein | 1,69 | 2,51 |
| Methionin | 1,95 | 0,73 |
| Tryptophan | 1,52 | 1,40 |
| Asparaginsäure | 11,26 | 0,63 |
| Threonin | 5,21 | 0,54 |
| Serin | 7,61 | 5,67 |
| Glutaminsäure | 9,76 | 0,65 |
| Prolin | 5,66 | 6,87 |
| Glycin | 7,76 | 0,55 |
| Alanin | 8,96 | 0,71 |
| Valin | 6,64 | 0,21 |
| Isoleucin | 4,05 | 1,75 |
| Leucin | 7,92 | 0,18 |
| Tyrosin | 3,46 | 1,02 |
| Phenylalanin | 3,71 | 0,95 |
| Histidin | 2,43 | 0,29 |
| Lysin | 4,84 | 0,29 |
| Arginin | 5,52 | 10,89 |

Zur Erläuterung der kennzeichnenden Merkmale des Gewebsproteins sei folgendes ausgeführt:

Die elektrophoretische Beweglichkeit wurde mit dem Gerät Microzone R 200 von Beckman Instruments auf Zelluloseazetatfolien (Firma Sartorius) unter Verwendung von Natriumdiäthylbarbiturat-Puffer, pH 8,6, bestimmt.

Der isoelektrische Punkt wurde mit einer Säule (440 ml) der Firma LKB, Stockholm, ermittelt. Das Ampholin®-Gemisch hatte einen pH-Bereich von 4,0 bis 6,0.

Der Sedimentationskoeffizient wurde in einer analytischen Ultrazentrifuge der Firma Beckman (Spinco-Apparatur, Modell E) bei 60.000 UpM in Doppelsektorzellen mit Hilfe der UV-Scanner-Technik bei 280 nm bestimmt. Als Lösungsmittel diente ein 0,05 mol/l Phosphatpuffer (pH 6,8), der 0,2 mol/l NaCl enthielt. Die Proteinkonzentration wurde auf eine optische Dichte von etwa 3 eingestellt. Der Sedimentationskoeffizient wurde auf die Basis von Wasser bei 20°C umgerechnet.

Zur Bestimmung der Molekulargewichte im SDS-haltigen Polyacrylamidgel wurden Gele mit 7,5 oder 10 g/100 ml Polyacrylamid (PAA) verwendet, die 0,1 g/100 ml Natriumdodecylsulfat (SDS) enthielten. Zur Bestimmung des nativen Molekulargewichts wurde $MP_1$, in Wasser oder in verdünntem (0,01 mol/l) Trispuffer gelöst, aufgetragen. Zur Bestimmung des Molekulargewichts der Untereinheiten ist die Substanz vor dem Auftragen in 1 g/100 ml SDS-Lösung 10 Minuten auf 60°C erhitzt worden. Als Vergleichssubstanzen dienten Transferrin, Humanalbumin und dessen Aggregate.

Zur Bestimmung des Extinktionskoeffizienten wurde 1 mg Substanz in destilliertem Wasser zu 1 ml Lösung gelöst.

Die Kohlenhydrate wurden wie folgt bestimmt:
Nach Hydrolyse der glykosidischen Bindungen wurden die freigesetzten Neutralzucker als Boratkomplexe über eine Anionenaustauschersäule getrennt (Y.C. Lee et al., Anal. Biochem. 27 (1969), 567), im Eluat durch Zumischung von Cu(I)-bicinchoninatreagenz (K. Mopper und M. Gindler, Anal. Biochem. 56 (1973), 440) angefärbt und unter Verwendung von Rhamnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, Vol. VI (1963), 463-465) ermittelt worden.

Die Aminosäurenanalyse wurde nach S. Moore, D.H. Spackman, W.H. Stein, Anal. Chem. 30 (1958), 1185, unter Verwendung des Flüssigkeitschromatographen Multichrom B der Firma Beckman durchgeführt. Cystin wurde nach Oxydation der Proteine mit Perameisensäure (S. Moore et al., Anal. Chem. 30 (1958), 1185) und nachfolgender Chromatographie (S. Moore, J. Biol. Chem. 238 (1963), 235) als Cysteinsäure bestimmt. Der Tryptophangehalt ist direkt photometrisch nach H. Edelhoch, Biochemistry 6 (1967), 1948, ermittelt worden.

Gegenstand der Erfindung ist weiter ein Verfahren zur Gewinnung des membran-assoziierten Plazentaproteins $MP_1$, dadurch gekennzeichnet, daß man

1. ausgewachsene menschliche Plazenten, wie sie bei der Entbindung anfallen, zerkleinert, mit physiologischer Salzlösung wäscht, bis alle löslichen Bestandteile entfernt sind und den so gewonnenen Geweberückstand mit einer 0,5 - 5 g eines nicht-ionischen Detergenzes auf 100 ml enthaltenden Lösung, vorzugsweise mit einer 2 ml/100 ml Polyethylenglycol-p-isooctylphenylether enthaltenden Lösung, extrahiert, den Überstand abtrennt, gegen Wasser oder Pufferlösung dialysiert und einengt;

2. die in der erhaltenen Lösung enthaltenen solubilisierten Gewebsantigene der Plazenta durch eine Immunadsorption weiter anreichert; und

3. die nach der Immunadsorption an Antigenen angereicherte Lösung durch eine Gelfiltration auftrennt und das Protein $MP_1$ in reiner Form gewinnt.

Zum Nachweis und zur Bestimmung des $MP_1$, etwa in einer Fraktion aus einer Trennoperation, können neben den angegebenen Parametern auch immunchemische Methoden dienen, da $MP_1$ antigene Eigenschaften hat. Bei der Immunisierung von Tieren mit diesem Protein werden spezifische
Antikörper gebildet.

Die in dem Verfahren zur Gewinnung des $MP_1$ gebrauchten
Antikörper werden durch Immunisieren von Kaninchen mit
der mittels Polyethylenglycol-p-isooctylphenylether solubilisierten Proteinfraktion erhalten.

Mit Hilfe des nach der vorliegenden Anmeldung gewonnenen
gereinigten $MP_1$ lassen sich durch Immunisieren von Tieren nach bekannten Methoden monospezifische Antiseren
herstellen.

Ein solches Antiserum kann einerseits zum immunologischen Nachweis des $MP_1$, andererseits zur Herstellung eines Immunadsorbens dienen, das zur Anreicherung und
Isolierung von $MP_1$ eingesetzt werden kann.

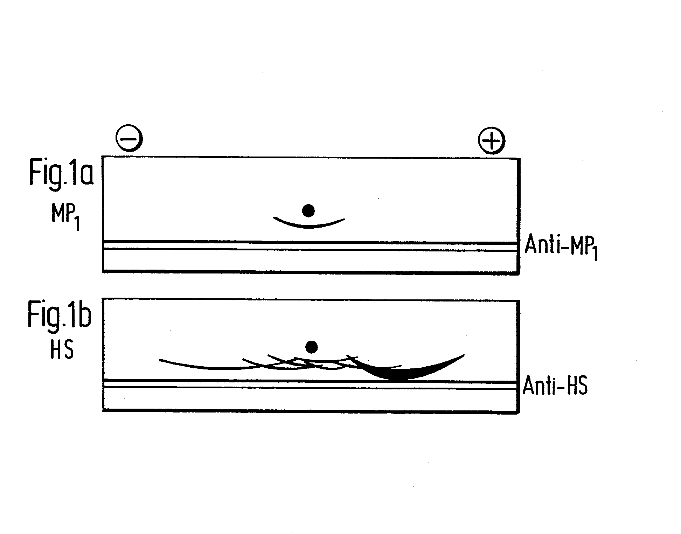

Abbildung 1a zeigt die immunologische Reaktion von $MP_1$
mit einem spezifischen Antiserum vom Kaninchen nach Auftrennung im elektrischen Feld in agar-haltigem Gel.

Abbildung 1b bringt zum Vergleich dazu die Auftrennung
der Proteine des Serums, sichtbar gemacht durch deren
Immunreaktion mit einem Antiserum vom Kaninchen gegen
Humanserum (HS).

Zum immunologischen Nachweis von $MP_1$ können auch die
Geldiffusionstechnik nach Ouchterlony (Schultze and
Heremans, Molecular Biology of Human Proteins, Vol. 1,
134) oder, wenn notwendig, auch empfindlichere Methoden
wie Radioimmunoassays oder Enzymimmunoassays herangezogen werden.

Der Nachweis und die Bestimmung von $MP_1$ haben diagnostische Bedeutung:

$MP_1$ scheint ein plazenta-"spezifisches" Protein zu sein. Die Konzentration solcher Proteine im Serum steigt im allgemeinen mit fortschreitender Schwangerschaft an. Sie lassen sich daher als Parameter zur Überwachung der Schwangerschaft verwenden. Plazentaspezifische Proteine sind andererseits aber auch vielfach bei Patienten mit Tumoren im Serum oder im Gewebe der Tumoren nachweisbar. Sie können daher bei solchen Erkrankungen als Marker zur Überwachung des Krankheitsverlaufs und zur Kontrolle einer Therapie verwendet werden.

$MP_1$ kann also verwendet werden, um Antiseren herzustellen, die dazu dienen können, $MP_1$ nachzuweisen und zu bestimmen.

Andererseits können Antikörper gegen $MP_1$ zur radioimmunologischen Lokalisation und zur immuntherapeutischen Behandlung von Tumoren, die $MP_1$ produzieren, eingesetzt werden.

Die Erfindung wird am nachstehenden Beispiel erläutert:

Beispiel

1.1 Zerkleinerung und Waschung der Plazenten

Zur Isolierung des membran-assoziierten Proteins $MP_1$ wurden reife menschliche Plazenten, wie sie bei einer Entbindung anfallen, verwendet. Die Plazenten wurden zunächst bei -20°C eingefroren, im gefrorenen Zustand mit einem Schneidmischer (Cutter) zerkleinert und in dieser Form bei -20°C bis zur Verwendung aufbewahrt. Durch Waschung mit physiologischer NaCl-Lösung wurden zunächst alle löslichen Gewebsproteine entfernt. Dazu wurden 500 g des zerkleinerten Plazentengewebes mit 700 ml NaCl-Lösung versetzt, kurz homogenisiert, dann mehrere Stunden bei 4°C gerührt und schließlich zentrifugiert. Der Überstand wurde verworfen, der Rückstand erneut mit 700 ml NaCl-Lösung mehrere Stunden gerührt und wieder zentrifugiert. Dieser Waschvorgang wurde insgesamt 6 mal wiederholt. Auf diese Weise wurde das Plazentagewebe von löslichen Bestandteilen weitgehend befreit.

1.2 Extraktion des Plazentagewebes mit Triton® X-100

Der Geweberückstand wurde dreimal mit je 700 ml einer Lösung von 2 ml Polyethylenglycol-p-isooctylphenylether (Triton®X-100) in 100 ml Wasser extrahiert, wobei jeweils 20 Stunden bei 4°C gerührt und dann abzentrifugiert wurde. Die Extrakte sind zunächst gegen Wasser und dann gegen einen 0,1 mol/l Tris-HCl-Puffer (pH 8,0), der 1 mol/l NaCl und 1g/l Natriumazid enthielt (Pufferlösung II), dialysiert worden. Die Dialysate wurden auf einem Ultrafilter (Fa. Amicon) unter Verwendung von PM-10 Membranen auf jeweils etwa 200 ml eingeengt. Die Extrakte aus dem Rückstand von 500 g Plazentagewebe enthielten im Durchschnitt insgesamt 22 mg $MP_1$ (Fraktion A).

2. Anreicherung von $MP_1$ durch Immunadsorption
-------------------------------------------------

2.1 Herstellung des Immunadsorbens

Durch Immunisieren von Kaninchen mit der solubilisierten Plazentaprotein-Fraktion A wurden polyvalente Antiseren hergestellt. Sie enthielten Antikörper sowohl gegen $MP_1$ als auch gegen andere mit Triton®X-100 löslich gemachte Gewebsantigene. 350ml eines solchen Antiserumpools wurden gegen 0,02 mol/l Phosphatpuffer (pH 7,0) dialysiert und zur Abtrennung der Immunglobuline mit dem gleichen Puffer an DEAE-Zellulose chromatographiert. Die Immunglobulinfraktion im Durchlauf (3,63 g Protein) wurde dann mit 363 g besonders gereinigter Agarose in Kugelform (Sepharose® von Pharmacia, Uppsala, Schweden), die mit 45,3 g Bromcyan aktiviert worden war, umgesetzt und so kovalent an einen Träger gebunden. Das Verfahren wurde beschrieben von Axen et al., Nature 214 (1967) 1302. Mit Hilfe eines auf diese Weise hergestellten Immunadsorbens konnte $MP_1$ zusammen mit anderen solubilisierten Antigenen aus der Plazentafraktion A weiter angereichert werden.

2.2 Durchführung der Immunadsorption

Das Immunadsorbens wurde in Pufferlösung II suspendiert, in eine Chromatographiesäule gefüllt (5,0 x 20 cm) und mit Pufferlösung II nachgespült. Dann wurde Fraktion A auf die Säule aufgetragen, wobei $MP_1$ und andere solubilisierte Antigene immunadsorptiv gebunden wurden. Die Säule wurde dann gründlich mit Puffer II gespült. Anschließend sind die adsorbierten Proteine mit etwa 600 ml 6 mol/l Harnstoff-Lösung von der Säule eluiert worden. Die $MP_1$-haltigen Eluate wurden gegen Pufferlösung II dialysiert und mit einem Ultrafilter auf etwa 20 ml eingeengt. Ausbeute pro Adsorption etwa 5 - 10 mg

$MP_1$. Das Adsorbens in der Säule wurde unmittelbar nach der Elution der Proteine wieder mit Pufferlösung II neutralisiert und gründlich gewaschen. Es ist dann erneut zur immunadsorptiven Bindung solubilisierter Antigene eingesetzt worden.

3. Isolierung von $MP_1$ durch Gelfiltration
----------------------------------------

Die Abtrennung von $MP_1$ von den anderen immunadsorptiv gebundenen Antigenen gelang durch Gelfiltration an Acrylamidagarose AcA-34 (LKB, Stockholm). Dazu wurden 10 durch Immunadsorption gewonnene Fraktionen vereinigt, auf 60 ml eingeengt und unter Verwendung der Pufferlösung II an einer AcA-34 Säule (5 x 110 cm) chromatographiert. $MP_1$ wurde dabei deutlich von den anderen solubilisierten Membranantigenen abgetrennt, die zur Hauptsache ein höheres Molekulargewicht (über 200.000) besitzen und daher schneller als $MP_1$ durch die Säule wandern, oder ein viel kleineres Molekulargewicht haben und erst nach dem $MP_1$ von der Säule eluiert werden. Die Fraktionen, welche die Hauptmenge $MP_1$ enthielten und frei von den anderen Antigenen waren, wurden vereinigt, gegen Wasser dialysiert und lyophilisiert. Die Ausbeute betrug etwa 15 mg $MP_1$.

Patentansprüche

1. Protein MP$_1$, gekennzeichnet durch

a) eine elektrophoretische Beweglichkeit im Bereich zwischen der von $\alpha_2$ und $\beta_1$ Globulinen;

b) einen isoelektrischen Punkt von $4,75 \pm 0,15$;

c) einen Sedimentationskoeffizienten $S^\circ_{20,w}$ von $6,65 \pm 0,15$ S;

d) ein im Natriumdodecylsulfat (SDS)-haltigen Polyacrylamidgel bestimmtes Molekulargewicht von $180.000 \pm 20.000$, wobei die Moleküle von MP$_1$ aus anscheinend identischen Untereinheiten, die ein Molekulargewicht von $90.000 \pm 10.000$ haben und nicht-kovalent zusammengehalten werden, aufgebaut sind;

e) einen Extinktionskoeffizienten $E^{1\%}_{1cm}$ (280 nm) von $8,2 \pm 0,4$;

f) einen Kohlenhydratanteil von $9,6 \pm 2,5\%$ (Mannose $2,2 \pm 0,5\%$, Galaktose $1,7 \pm 0,4\%$, Fucose $0,4 \pm 0,2\%$, N-Acetyl-Glukosamin $3,2 \pm 0,8\%$, N-Acetyl-Neuraminsäure $2,1 \pm 0,6\%$) und

g) die folgende Aminosäurenzusammensetzung:

| Aminosäure | Reste pro 100 Reste (Mol %) | Variationskoeffizient |
|---|---|---|
| Cystein | 1,69 | 2,51 |
| Methionin | 1,95 | 0,73 |
| Tryptophan | 1,52 | 1,40 |
| Asparaginsäure | 11,26 | 0,63 |
| Threonin | 5,21 | 0,54 |
| Serin | 7,61 | 5,67 |
| Glutaminsäure | 9,76 | 0,65 |
| Prolin | 5,66 | 6,87 |
| Glycin | 7,76 | 0,55 |
| Alanin | 8,96 | 0,71 |
| Valin | 6,64 | 0,21 |

| | | |
|---|---|---|
| Isoleucin | 4,05 | 1,75 |
| Leucin | 7,92 | 0,18 |
| Tyrosin | 3,46 | 1,02 |
| Phenylalanin | 3,71 | 0,95 |
| Histidin | 2,43 | 0,29 |
| Lysin | 4,84 | 0,29 |
| Arginin | 5,52 | 10,89. |

2. Verfahren zur Gewinnung des Protein $MP_1$, das

a) eine elektrophoretische Beweglichkeit im Bereich zwischen der von $\alpha_2$ und $\beta_1$ Globulinen;

b) einen isoelektrischen Punkt von $4,75 \pm 0,15$;·

c) einen Sedimentationskoeffizienten $S^\circ_{20,w}$ von $6,65 \pm 0,15$ S;

d) ein im Natriumdodecylsulfat (SDS)-haltigen Polyacrylamidgel bestimmtes Molekulargewicht von $180.000 \pm 20.000$, wobei die Moleküle von $MP_1$ aus anscheinend identischen Untereinheiten, die ein Molekulargewicht von $90.000 \pm 10.000$ haben und nicht-kovalent zusammengehalten werden, aufgebaut sind;

e) einen Extinktionskoeffizienten $E^{1\%}_{1cm}$ (280 nm) von $8,2 \pm 0,4$;

f) einen Kohlenhydratanteil von $9,6 \pm 2,5\%$ (Mannose $2,2 \pm 0,5\%$, Galaktose $1,7 \pm 0,4\%$, Fucose $0,4 \pm 0,2\%$, N-Acetyl-Glukosamin $3,2 \pm 0,8\%$, N-Acetyl-Neuraminsäure $2,1 \pm 0,6\%$) und

g) die folgende Aminosäurenzusammensetzung:

| Aminosäure | Reste pro 100 Reste (Mol %) | Variations-koeffizient |
|---|---|---|
| Cystein | 1,69 | 2,51 |
| Methionin | 1,95 | 0,73 |
| Tryptophan | 1,52 | 1,40 |
| Asparaginsäure | 11,26 | 0,63 |
| Threonin | 5,21 | 0,54 |
| Serin | 7,61 | 5,67 |

| | | |
|---|---|---|
| Glutaminsäure | 9,76 | 0,65 |
| Prolin | 5,66 | 6,87 |
| Glycin | 7,76 | 0,55 |
| Alanin | 8,96 | 0,71 |
| Valin | 6,64 | 0,21 |
| Isoleucin | 4,05 | 1,75 |
| Leucin | 7,92 | 0,18 |
| Tyrosin | 3,46 | 1,02 |
| Phenylalanin | 3,71 | 0,95 |
| Histidin | 2,43 | 0,29 |
| Lysin | 4,84 | 0,29 |
| Arginin | 5,52 | 10,89 |

hat, dadurch gekennzeichnet, daß man

1. ausgewachsene menschliche Plazenten, wie sie bei der Entbindung anfallen, zerkleinert, mit physiologischer Salzlösung wäscht, bis alle löslichen Bestandteile entfernt sind und den so gewonnenen Geweberückstand mit einer 0,5-5 g eines nicht-ionischen Detergenzes auf 100 ml enthaltenden Lösung, vorzugsweise mit einer 2 ml/100 ml Polyethylenglycol-p-jsooctylphenylether enthaltenden Lösung, extrahiert, den Überstand abtrennt, gegen Wasser oder Pufferlösung dialysiert und einengt;

2. die in der erhaltenen Lösung enthaltenen solubilisierten Gewebsantigene der Plazenta durch eine Immunadsorption weiter anreichert; und

3. die nach der Immunadsorption an Antigenen angereicherte Lösung durch eine Gelfiltration auftrennt und das Protein $MP_1$ in reiner Form gewinnt.

3. Verwendung des Proteins nach Anspruch 1 zur Herstellung von Antikörpern gegen $MP_1$ zum Nachweis und zur Bestimmung von $MP_1$ sowie zur radioimmunologischen Lokalisation und zur immuntherapeutischen Behandlung von Tumoren, die $MP_1$ produzieren.

0125514

Fig.1a
MP$_1$

Anti-MP$_1$

Fig.1b
HS

Anti-HS